# EUROPÄISCHE PATENTSCHRIFT

(11) **EP 3 713 526 B1**
(45) Veröffentlichungstag und Bekanntmachung des Hinweises auf die Patenterteilung: **10.11.2021**
(21) Anmeldenummer: 18808320.8
(22) Anmeldetag: 23.11.2018
(51) Int. Cl.: A61F 5/01

(54) **EXTREMITÄTENORTHESE, INSBESONDERE KNIEORTHESE UND ELLENBOGENORTHESE**
LIMB ORTHOSIS, IN PARTICULAR KNEE BRACE AND ELBOW BRACE
ORTHÈSE D'EXTRÉMITÉ, EN PARTICULIER ORTHÈSE DE GENOU ET ORTHÈSE DE COUDE

(30) Priorität: 23.11.2017 DE 102017220968
(43) Veröffentlichungstag der Anmeldung: 30.09.2020
(73) Patentinhaber: Bauerfeind AG, 07937 Zeulenroda-Triebes (DE)
(72) Erfinder: HEBENSTREIT, Sandro, 07937 Zeulenroda-Triebes (DE); STIER, Gerald, 07957 Langenwetzendorf (DE); BAUERFEIND, Hans B., 07937 Zeulenroda-Triebes (DE)
(74) Vertreter: Wohlfahrt, Jan Günther
(86) Internationale Anmeldenummer: PCT/EP2018/082346
(87) Internationale Veröffentlichungsnummer: WO 2019/101910

(56) Entgegenhaltungen:
- WO-A1-2014/043695
- US-A1- 2015 305 910
- US-A1- 2016 367 391

## Beschreibung

Die vorliegende Erfindung betrifft eine Orthese für Extremitäten, insbesondere eine Knieorthese oder eine Ellenbogenorthese, umfassend ein bevorzugt über ein Kopplungselement gekoppeltes Gurtsystem, dadurch gekennzeichnet, dass ein Gurt als erster Abschnitt des Gurtsystems so geführt ist, dass der Gurt sich in einem ersten Kreuzungsbereich kreuzt und dass ein Gurt als zweiter Abschnitt des Gurtsystems so geführt ist, dass der Gurt sich in einem zweiten Kreuzungsbereich kreuzt, wie in den Ansprüchen beschrieben.

Die vorliegende Erfindung betrifft insbesondere auch eine Orthese für Extremitäten, insbesondere eine Knieorthese oder eine Ellenbogenorthese, umfassend ein Trägerelement mit einem oberen Teilbereich, einem mittleren Teilbereich und einem unteren Teilbereich und umfassend ein an das Trägerelement über ein Kopplungselement gekoppeltes Gurtsystem, wobei ein Gurt als erster Abschnitt des Gurtsystems um den oberen Teilbereich des Trägerelements so geführt ist, dass der Gurt sich in einem ersten Kreuzungsbereich kreuzt und dass ein Gurt als zweiter Abschnitt des Gurtsystems um den unteren Teilbereich des Trägerelements so geführt ist, dass der Gurt sich in einem zweiten Kreuzungsbereich kreuzt, wie in den Ansprüchen beschrieben.

Orthesen für Extremitäten, auch Extremitätenorthese genannt, insbesondere Knieorthesen zur passiven und aktiven Stabilisierung des Kniegelenks sind aus dem Stand der Technik bekannt. Solche Knieorthesen weisen häufig ein Trägerelement in Form einer Bandage auf, auf das Stabilisierungselemente wie Gurte, Stäbe oder Gelenkschienen montiert sind, wie es beispielsweise in der DE 200 05 366 U1 gezeigt ist.

Solche Knieorthesen werden beispielsweise bei einer Kniearthrose oder nach Verletzung des Innenminiskus eingesetzt, um den betroffenen Bereich zu entlasten und zu stabilisieren. Knieorthesen weisen dabei häufig Gurte auf, mit denen die Knieorthese eingestellt werden, also an die Beinform und/oder auf den Wirkungsbereich angepasst werden kann. Spezifisch geführte Gurte sind beispielsweise aus der EP 2 612 626 A2, der DE 40 136 93 A1 und der DE 198 44 545 A1 bekannt.

US2015305910 offenbart eine Knieorthese mit einem um den Oberschenkel befestigten Kompressionssystem und einem um den Unterschenkel befestigten Kompressionssystem, wobei seitliche Gelenkanordnungen beide Kompressionssysteme verbinden. Ferner umfasst die Knieorthese mindestens zwei Gurte, wobei beide Gurte sowohl mit dem oberen als auch dem unteren Teil der Knieorthese gekoppelt sind und beide hinter dem Knie gekreuzt werden. In D1 soll ferner die Kopplung des ersten sowie des zweiten Gurtes durch ein Kopplungselement im oberen Abschnitt der Knieorthese offenbart werden. US2016367391 offenbart eine Knieorthese mit einem elastischen gekreuzten Gurt, welcher ebenso einen oberen und einen unteren Abschnitt aufweist, welcher im oberen und unteren Abschnitt sowie hinter dem Knie gekreuzt werden. Das technische Problem der vorliegenden Erfindung ist es, eine verbesserte Orthese für Extremitäten, insbesondere Knieorthese bereitzustellen. Insbesondere soll die Orthese für Extremitäten, insbesondere Knieorthese besonders gut zur Behandlung der medialen Gonarthrose geeignet sein und dabei das mediale oder innere Kompartiment des Femorotibial-Gelenks entlasten und somit zu einer verbesserten Schmerzreduzierung führen. Auch soll die Knieorthese dabei sicher und fest am Bein sitzen.

Die vorliegende Erfindung löst das technische Problem durch eine Orthese für eine Extremität, insbesondere Knieorthese nach Anspruch 1.

Unter einer Extremität wird in der vorliegenden Erfindung insbesondere ein Bein oder ein Arm verstanden, wobei auch der Hüftbereich zur Extremität gezählt werden kann. Im Zusammenhang mit der vorliegenden Erfindung wird unter einer Orthese für Extremitäten aber neben insbesondere einer Beinorthese, Knieorthese, Armorthese oder Ellenbogenorthese beispielsweise auch eine Hüftorthese verstanden. Bevorzugt ist die Orthese für Extremitäten eine Knieorthese, Ellenbogenorthese oder Hüftorthese.

Bei der Orthese für Extremitäten, auch Extremitätenorthese genannt, kann es sich insbesondere um eine Knieorthese oder einer Ellenbogenorthese handeln. Bevorzugt handelt es sich um eine Knieorthese.

Die vorliegende Erfindung betrifft eine Orthese für Extremitäten, insbesondere Knieorthese, umfassend ein Gurtsystem, dadurch gekennzeichnet, dass ein Gurt als erster Abschnitt des Gurtsystems so geführt ist, dass der Gurt sich in einem ersten Kreuzungsbereich kreuzt und dass ein Gurt als zweiter Abschnitt des Gurtsystems so geführt ist, dass der Gurt sich in einem zweiten Kreuzungsbereich kreuzt, wie in den Ansprüchen beschrieben.

Die vorliegende Erfindung betrifft insbesondere eine Extremitätenorthese, umfassend ein Gurtsystem, dadurch gekennzeichnet, dass ein Gurt als erster Abschnitt des Gurtsystems so geführt ist, dass der Gurt sich in einem ersten Kreuzungsbereich kreuzt und dass ein Gurt als zweiter Abschnitt des Gurtsystems so geführt ist, dass der Gurt sich in einem zweiten Kreuzungsbereich kreuzt und umfassend ein Kopplungselement, an dem der erste Abschnitt des Gurtsystems und der zweite Abschnitt des Gurtsystems gekoppelt sind, wobei bei der angelegten Extremitätenorthese der erste Abschnitt und der zweite Abschnitt des Gurtsystems so um die Extremität geführt sind, dass der erste Abschnitt und der zweite Abschnitt des Gurtsystems die Extremität umschlingt und sich dabei mindestens einmal kreuzt, wie in den Ansprüchen beschrieben. Wenn der erste

Abschnitt und/oder der zweite Abschnitt des Gurtsystems die Extremität umschlingt, also mindestens einmal ganz um die Extremität herumgeführt ist, sind bevorzugt beide Enden des ersten Abschnitts und/oder des zweiten Abschnitts mit dem Kopplungselement verbunden.

Der erste Abschnitt des Gurtsystems liegt bei einer Knieorthese im angelegten Zustand am Oberschenkel an, der zweite Abschnitt des Gurtsystems liegt im angelegten Zustand am Unterschenkel an.

Der erste Abschnitt des Gurtsystems umschlingt bei einer Knieorthese bevorzugt im angelegten Zustand den Oberschenkel, der zweite Abschnitt des Gurtsystems umschlingt im angelegten Zustand bevorzugt den Unterschenkel. Der erste Abschnitt des Gurtsystems liegt bei einer Ellenbogenorthese im angelegten Zustand am Oberarm an, der zweite Abschnitt des Gurtsystems liegt im angelegten Zustand am Unterarm an.

Der erste Abschnitt des Gurtsystems umschlingt bei einer Ellenbogenorthese bevorzugt im angelegten Zustand den Oberarm, der zweite Abschnitt des Gurtsystems umschlingt im angelegten Zustand bevorzugt den Unterarm.

Bevorzugt umfasst die Extremitätenorthese, bevorzugt Knieorthese ein Kopplungselement, an dem der erste Abschnitt des Gurtsystems und der zweite Abschnitt des Gurtsystems gekoppelt sind. Bevorzugt liegt das Kopplungsclcmcnt im angelegten Zustand auf Höhe des Knies am Bein an. Bevorzugt ist das Kopplungselement im angezogenen Zustand der Knieorthese auf der Außenseite des Beins positioniert.

Das Kopplungselement kann im angezogenen Zustand der erfindungsgemäßen Orthese je nach Bedarf auf der Außenseite oder der Innenseite der Extremität positioniert sein. Beispielsweise kann bei der Verwendung bei einem "O-Bein" das Kopplungselement auf der Außenseite des Beins positioniert sein und bei der Verwendung bei einem "X-Bein" das Kopplungselement auf der Innenseite des Beins positioniert sein.

Das Gurtsystem bildet also zwei Kreuzungsbereiche, von denen einer oberhalb des Knies liegt und der andere unterhalb des Knies. Es zeigte sich überraschenderweise, dass diese beiden Kreuzungsbereiche in vorteilhafter Weise dazu führen, dass die Knieorthese am Oberschenkel, wie auch am Unterschenkel positionsstabil sitzt. Eine solche Positionsstabilität ist eine wesentliche Voraussetzung für die konstante hohe Wirksamkeit der Knieorthese. Darüber hinaus können die beiden Kreuzungsbereiche bevorzugt in vorteilhafter Weise als Gegenlager für die Scheitelpunktkraft auf der Außenseite des Knies dienen. Es kann auch in vorteilhafter Weise durch die beiden Kreuzungsbereiche auf eine Abstandsschiene auf der Innenseite der Knieorthese verzichtet werden, wenn die Kreuzungsbereiche in bevorzugter Ausführungsform auf der Innenseite des Beins zu liegen kommen und das Kopplungselement in bevorzugter Ausführungsform auf Höhe des Knies so positioniert ist, dass es auf der Außenseite des Beins zu liegen kommt. Bevorzugt sind die beiden Kreuzungsbereiche im angezogenen Zustand der Knieorthese auf der Innenseite des Beins positioniert.

Die erfindungsgemäße Orthese kann in vorteilhafter Weise zur Korrektur von Fehlstellungen an einem Gelenk genutzt werden, beispielsweise "X-Beine" oder "O-Beine". Die erfindungsgemäße Orthese kann aber auch in vorteilhafter Weise dazu dienen, die Gelenkbeugung oder die Gelenkstreckung zu limitieren.

Es zeigte sich, dass der erfindungsgemäße Aufbau der Knieorthese dazu führt, dass die Knieorthese angenehmer zu tragen ist, wobei sie dabei das Knie mindestens so gut entlastet wie Knieorthesen aus dem Stande der Technik. Dabei ist die erfindungsgemäße Knieorthese weniger rigide als solche aus dem Stand der Technik.

Bei einer Ellenbogenorthese umfasst diese entsprechend ein Kopplungselement, an dem der erste Abschnitt des Gurtsystems und der zweite Abschnitt des Gurtsystems gekoppelt sind. Bevorzugt liegt das Kopplungselement im angelegten Zustand auf Höhe des Ellenbogens am Arm an. Bevorzugt ist das Kopplungselement im angezogenen Zustand der Ellenbogenorthese auf der Außenseite des Arms positioniert.

Bevorzugt sind bei der Orthese für Extremitäten, insbesondere Knieorthese, der erste Gurtabschnitt und/oder der zweite Gurtabschnitt als sich kreuzende Schlinge um das Bein oder den Arm geführt. Bevorzugt sind bei der Orthese für Extremitäten, insbesondere Knieorthese, der erste Gurtabschnitt und der zweite Gurtabschnitt jeweils als sich kreuzende Schlinge um das Bein oder den Arm geführt.

Die erfindungsgemäß bevorzugte Führung der Gurtabschnitte in Schlingenform kann die Wirkung des Gurtsystems deutlich unterstützen.

Bevorzugt ist also der erste Abschnitt und/oder der zweite Abschnitt des Gurtsystems so um eine Extremität, insbesondere ein Bein, geführt, dass der Gurtabschnitt die Extremität umschlingt und sich dabei mindestens einmal, bevorzugt einmal kreuzt. Bevorzugt sind dabei die Enden des ersten Abschnitts und/oder des zweite Abschnitts des Gurtsystems am Kopplungselement befestigt, insbesondere im mittleren Bereich der Orthese für Extremitäten, insbesondere Knieorthese.

Bevorzugt ist also eine Extremitätenorthese, wobei bei der angelegten Extremitätenorthese der erste Abschnitt und/oder der zweite Abschnitt des Gurtsystems so um die Extremität, insbesondere ein Bein, geführt sind, dass der erste Abschnitt und/oder der zweite Abschnitt des Gurtsystems die Extremität umschlingt und sich dabei einmal kreuzt, also wobei der erste Abschnitt und/oder der zweite Abschnitt des Gurtsystems jeweils in Form einer 8 um die Extremität geführt sind.

Bei der Orthese für Extremitäten, insbesondere Knieorthese kann zusätzlich ein Trägerelement vorhanden sein, beispielsweise eine Bandage, es kann aber durch den Verlauf des erfindungsgemäßen Gurtsystems auch auf das Trägerelement verzichtet werden.

Die vorliegende Erfindung betrifft also auch eine Orthese für Extremitäten, insbesondere Knieorthese, umfassend ein Trägerelement mit einem oberen Teilbereich, einem mittleren Teilbereich und einem unteren Teilbereich und umfassend ein an das Trägerelement über ein Kopplungselement gekoppeltes Gurtsystem, dadurch gekennzeichnet, dass ein Gurt als erster Abschnitt des Gurtsystems um den oberen Teilbereich des Trägerelements so geführt ist, dass der Gurt sich in einem ersten Kreuzungsbereich kreuzt und dass ein Gurt als zweiter Abschnitt des Gurtsystems um den unteren Teilbereich des Trägerelements so geführt ist, dass der Gurt sich in einem zweiten Kreuzungsbereich kreuzt.

Der obere Teilbereich des Trägerelements liegt im angelegten Zustand am Oberschenkel oder Oberarm an, der untere Teilbereich des Trägerelements liegt im angelegten Zustand am Unterschenkel oder Unterarm an und der mittlere Teilbereich des Trägerelements liegt im angelegten Zustand im Bereich des Knies am Bein oder des Ellenbogens am Arm an.

Die Orthese für Extremitäten, insbesondere Knieorthese weist also ein Gurtsystem auf, das um das Bein oder den Arm des Trägers, also bevorzugt auch um das Trägerelement, in Form einer doppelten Acht geführt ist, wobei insbesondere die erste, obere Acht am oberen Teilbereich des Trägerelements geführt ist, der im angelegten Zustand am Oberschenkel oder Oberarm anliegt und die zweite untere Acht um den unteren, zweiten Teilbereich des Trägerelements geführt ist, der im angelegten Zustand am Unterschenkel oder Unterarm anliegt.

Die Gurte des Gurtsystems können bevorzugt auf dem Trägerelement, im Trägerelement oder unter dem Trägerelement geführt werden. Es kann auch auf ein Trägerelement verzichtet werden.

In einer bevorzugten Ausführungsform ist das Kopplungselement am mittleren Teilbereich an dem Trägerelement befestigt. Bevorzugt ist das Kopplungselement am mittleren Teilbereich an dem Trägerelement so befestigt, dass es im angezogenen Zustand der Orthese für Extremitäten auf der Außenseite des Beins oder Arms positioniert ist. Bevorzugt ist das Kopplungselement am mittleren Teilbereich an dem Trägerelement so befestigt, dass es im angezogenen Zustand der Knieorthese auf der Außenseite des Beins positioniert ist.

Bevorzugt sind die beiden Kreuzungsbereiche am Trägerelement so befestigt, dass sie im angezogenen Zustand der Orthese für Extremitäten auf der Innenseite der Extremität positioniert sind.

Bevorzugt sind die beiden Kreuzungsbereiche am Trägerelement so befestigt, dass sie im angezogenen Zustand der Knieorthese auf der Innenseite des Beins positioniert sind.

In einer bevorzugten Ausführungsform ist das Trägerelement eine Bandage. Geeignete Bandagen sind dem Fachmann bekannt. Insbesondere kann es sich bei dem Trägerelement um eine Bandage in Form einer Maschenware, beispielsweise eines Gestricks oder eines Gewirks handeln. Bevorzugt ist die Bandage eine textile Bandage.

In einer bevorzugten Ausführungsform weist das Gurtsystem ein erstes Kreuzungselement auf, in dem der Gurt im ersten Kreuzungsbereich geführt wird und weist ein zweites Kreuzungselement auf, in dem der Gurt im zweiten Kreuzungsbereich geführt wird. Die Kreuzungselemente können in vorteilhafter Weise als Führungsschienen für die Gurte dienen. Bevorzugt ist das erste Kreuzungselement reversibel an dem oberen Teilbereich des Trägerelements befestigbar und das zweite Kreuzungselement reversibel an dem unteren Teilbereich des Trägerelement befestigbar.

Die Kreuzungselemente können in vorteilhafter Weise als Führungsschienen für die Gurte dienen, um bevorzugt auch den Kreuzungsbereich reversibel auf dem Trägerelement fixieren zu können. Dies erlaubt in vorteilhafter Weise eine exakte und dennoch flexible Positionierung der beiden Kreuzungsbereiche, sodass diese besonders gut positionsstabil sitzen und punktgenau wirken können.

In einer bevorzugten Ausführungsform sind das erste und das zweite Kreuzungselement über eine Klettverbindung an verschiedenen Stellen des Trägerelements reversibel befestigbar.

Durch eine Klettverbindung können die Kreuzungselemente auf einfache und sichere Weise an dem Trägerelement reversibel befestigt sein. Dabei kann beispielsweise das Trägerelement Flausch- oder Klettbereiche aufweisen, auf denen ein Kreuzungselement positioniert und befestigt werden kann. Dabei hat das Kreuzungselement auf seiner Unterseite bevorzugt eine Klett- oder Flauschoberfläche. Mit einem entsprechenden Flausch- oder Klettbereich auf dem Trägerelement kann in vorteilhafter Weise eine Fläche bereitgestellt werden, in der das Kreuzungselement frei positionierbar ist und gleichzeitig kann durch die Positionierung, Dimensionierung und Form des Klett- oder Flauschbereichs auf dem Trägerelement für die Wirksamkeit sinnvolle Bereiche vorgegeben werden, auf denen die Kreuzungselemente befestigt werden sollten.

In einer bevorzugten Ausführungsform wird der erste Abschnitt des Gurtsystems durch einen ersten Gurt gebildet und der zweite Abschnitt des Gurtsystems wird durch einen zweiten Gurt gebildet. In dieser bevorzugten Ausführungsform wird also die erste obere Acht durch einen ersten Gurt und die zweite untere Acht durch einen zweiten Gurt gebildet. Dabei sind bevorzugt beide Gurte an dem Kopplungselement befestigt.

In einer bevorzugten Ausführungsform ist das Gurtsystem an dem Kopplungselement beweglich befestigt. Die bewegliche Befestigung kann beispielsweise in Form einer drehbaren Aufhängung der Gurte an dem Kopplungselement erfolgen. Dadurch können die Gurte sich auch im Bereich des Kopplungselements in ihrem Verlauf am Bein, am Arm oder an dem Trägerelement ändern, wenn die Positionierung der Kreuzungsbereiche verändert wird.

Die Gurte des Gurtsystems können entweder aus Flachgurten, insbesondere im Wesentlichen unelastischen Flachgurten bestehen oder aus Seilen bzw. Schnüren bestehen, die bevorzugt in Tunneln verlaufen, die z.B. aus Flachbändern gebildet werden. Dies hat den Vorteil, dass die Gurte beim Spannen oder Entspannen keine Falten werfen.

In einer bevorzugten Ausführungsform weist das Kopplungselement ein Spannelement zum Spannen des Gurtes des ersten Abschnitts des Gurtsystems und/oder zum Spannen des Gurtes des zweiten Abschnitts des Gurtsystems auf. In vorteilhafter Weise ist also vorgesehen, dass die beiden Gurte zusammen oder getrennt gespannt werden können, sodass sie zum einen einen festen Sitz der Orthese für Extremitäten an der Extremität, insbesondere der Knieorthese an dem Bein ermöglichen und zum anderen eine gute Positionierung und einen guten Sitz einer bevorzugten Gelenkschiene ermöglichen.

In einer bevorzugten Ausführungsform ist das Spannelement als Drehknopf ausgebildet, mit dem beide Gurte gleichzeitig gespannt werden können. Bevorzugt werden die Gurte also durch Drehen eines Knopfes gespannt, beispielsweise indem durch den Drehknopf Schnüre aufgewickelt werden, die mit den Gurten verbunden sind oder die Gurte als Schnüre ausgebildet sind, die mit Hilfe des Drehknopfs aufgewickelt werden. Es ist also bevorzugt vorgesehen, dass die beiden Gurte gleichzeitig durch ein Spannelement gespannt werden, sodass die beiden Gurte die entsprechend gleiche Spannkraft haben und somit die beiden Gurte als Gurtsystem zu einem einzigen Zugsystem zusammengeführt sind und nur ein Arbeitsschritt zum Spannen beider Gurte notwendig ist. Bevorzugt ist das Spannsystem am Kopplungselement oder wie das Kopplungselement selbst am mittleren Teilbereich an dem Trägerelement befestigt. Natürlich ist das Spannelement so ausgebildet, dass die Gurte auch wieder entspannt werden können. Beispielsweise durch eine Rasterung im Drehknopf können bevorzugt verschiedene Spannungsgrade eingestellt werden, so dass in vorteilhafter Weise eine spezifische Krafteinstellung möglich ist.

Die Erfindung zeichnet sich also insbesondere durch die Führung der Gurte als doppelte 8 aus, wobei die Gurte mittels eines zentralen Spannelements gegeneinander verspannt und über doppelte Gurtkreuze symmetrisch zum oberen und unteren Ende der Orthese geführt werden, wo sie jeweils eine Schlinge bilden. Dabei sind bevorzugte Vorteile insbesondere ein symmetrischer Spannungseintrag und ein einziges Spannsystem zum Spannen der Gurte über die ganze Orthese. Auch kann das System mit oder ohne Trägerelement eingesetzt werden.

Durch die spezifische, symmetrische Gurtführung mit in sich geschlossener Schlingenbildung wird eine Positionskorrektur vom Oberschenkel oder Oberarm zum Unterschenkel oder Unterarm erreicht.

In einer bevorzugten Ausführungsform weist die Orthese für eine Extremität, insbesondere Knieorthese zusätzlich eine Gelenkschiene auf.

Gelenkschienen sind meist so ausgeführt, dass nicht nur in der sagittalen Elemente die Kniebewegung, sondern auch in der Frontalebene eine Einstellung stattfinden kann. Dabei handelt es sich in der Frontalebene um einstellbare Scharniergelenke, die sich so nahe wie möglich am Krümmungsscheitel des Kniegelenks oder Ellenbogengelenks befinden, um so im Sinne einer Entlastung des inneren Kompartiments des Femortibialgelenks oder Ellenbogengelenks zu wirken. Die im Stand der Technik gezeigten zirkulär angeordneten Gurte wirken als Gegenlager oberhalb und unterhalb des Kniegelenks auf der gegenüberliegenden Seite der Gelenkschiene. Solche Gelenkschienen sind beispielsweise aus der WO 2007/145504 A1 oder der WO 2003/103547 A1 bekannt.

Es zeigte sich überraschenderweise, dass durch den doppelt gekreuzten Verlauf des erfindungsgemäßen Gurtsystems die Gelenkschiene besonders gut, insbesondere auf der Außenseite des Beins oder Arms an das Bein oder an den Arm und bevorzugt das Trägerelement positionsstabil angepresst wird, da die beiden bevorzugt innenliegenden Kreuzungsbereiche als Gegenkräfte zu dem Kopplungselement wirken, das bevorzugt an der Gelenkschiene befestigt ist. Somit kann in bevorzugter Ausführungsform die Orthese für eine Extremität, insbesondere Knieorthese biomechanisch besonders gut nach dem Dreikräfteprinzip (zwei Ansatzhauptpunkte und ein entgegengesetzt wirkender Druckpunkt) wirken. Eine solche Wirkung basierend auf dem Dreikräfteprinzip ist in vorteilhafter Weise dazu geeignet, die Repositionierung von Ober- und Unterschenkel-Knochen beziehungsweise Ober- und Unterarm-Knochen über das Gelenk selbst zu unterstützen. Es kann damit bevorzugt der Gelenkspalt entgegen dem Krankheitsbild korrigiert werden und es kann dadurch zu einer deutlichen Schmerzreduktion kommen.

Bevorzugt ist die Gelenkschiene an der Orthese für eine Extremität, insbesondere Knieorthese so befestigt, dass sie auf der Außenseite des Beins oder Arms bei der angezogenen Orthese für eine Extremität, insbesondere Knieorthese positioniert ist. Bei Bedarf kann die Gelenkschiene aber auch auf der Innenseite des Beins oder Arms positioniert sein.

Bevorzugt ist die Gelenkschiene an dem Trägerelement so befestigt, dass sie auf der Außenseite des Beins oder Arms bei der angezogenen Orthese für eine Extremität, insbesondere Knieorthese positioniert ist.

In einer bevorzugten Ausführungsform weist die Gelenkschiene einen ersten Schienenabschnitt, einen zweiten Schienenabschnitt und einen dritten Schienenabschnitt auf, wobei der erste Schienenabschnitt und der zweite Schienenabschnitt jeweils durch ein Gelenk mit dem dritten Schienenabschnitt verbunden sind.

In einer bevorzugten Ausführungsform weist die Gelenkschiene einen ersten Schienenabschnitt im Bereich des oberen Teilbereichs des Trägerelements, einen zweiten Schienenabschnitt im Bereich des unteren Teilbereichs des Trägerelements und einen dritten Schienenabschnitt im Bereich des mittleren Teilbereichs des Trägerelements auf, wobei der erste Schienenabschnitt und der zweite Schienenabschnitt jeweils durch ein Gelenk mit dem dritten Schienenabschnitt verbunden sind.

Bevorzugt sind das Kopplungselement und entsprechend bevorzugt auch das Spannelement am dritten Schienenabschnitt im Bereich des mittleren Teilbereichs der Orthese für eine Extremität, insbesondere Knieorthese, bevorzugt des Trägerelements an der Gelenkschiene befestigt. Insbesondere kann auch der mittlere Schienenabschnitt das Kopplungselement bilden.

In einer bevorzugten Ausführungsform sind die Schienenabschnitte elastisch ausgestaltet.

In einer bevorzugten Ausführungsform wird der dritte Schienenabschnitt aus einem Federelement, insbesondere einer Blattfeder, gebildet.

In einer bevorzugten Ausführungsform sind die Gelenke monozentrische Gelenke. Die zwei monozentrischen Gelenke oberhalb und unterhalb des dritten, mittleren Schienenabschnitts ergeben zusammen eine Duo-Zentrik, die in vorteilhafter Weise dazu führt, dass eine Beugung des Knies weniger Stress im Kniegelenk erzeugt. Während der Beugung kann das Drehzentrum mehrfach von einem zum anderen Drehpunkt wechseln. In Kombination mit elastischen Schienen, insbesondere der Blattfeder, sorgt die Duo-Zentrik dafür, dass die Orthese sich in der Beugung nicht seitlich gegen die Extremitätenachse, insbesondere Beinachse verschiebt.

Die Ausbildung des dritten Schienenabschnitts aus einem Federelement, insbesondere aus einer Blattfeder, beispielsweise aus Federstahl, führt dazu, dass der erste Schienenabschnitt und der zweite Schienenabschnitt nicht nur in einer Achse rotieren, sondern auch in einer um 90° gedrehten Achse beweglich sind, und zwar bevorzugt in dem Bereich, in dem durch das Kopplungselement der mittlere Krafteinleitungspunkt des durch das Gurtsystem hergestellte Dreipunktsystems flexibel ist. Darüber hinaus führen die beiden Gelenke, die den ersten Schienenabschnitt beziehungsweise den zweiten Schienenabschnitt mit dem dritten Schienenabschnitt verbinden, dazu, dass sich die Gelenkschiene als solche viel besser an die Konturen des Körpers anpassen kann. Auch führt die Federung des dritten Schienenabschnitts vorteilhafterweise dazu, dass die Orthese für eine Extremität, insbesondere Knieorthese bei extremen Bewegungen etwas nachgeben kann und dabei den Benutzer vor einer übermäßig falschen Beinbewegung warnt.

Durch die Ausbildung des dritten Schienenabschnitts als Federelement wird in vorteilhafter Weise der mittlere Krafteinleitungspunkt federnd gelagert.

Die Ausführung des Gurtsystems, also die 8er Führung, ist so gestaltet das die Gurtkreuze nicht zur Kniemitte oder Ellenbogenmitte hinwandern können. Dabei fungiert die laterale Schiene als Abstandshalter für die beiden medialen Gurtkreuze. Damit wird eine optimale Positionsstabilität aller Funktionselemente zueinander gewährleistet. Außerdem bleibt die Orthese dadurch positionsstabil am Bein oder am Arm.

Die vorliegende Erfindung betrifft auch die Verwendung einer erfindungsgemäßen Orthese zur Behandlung von Leiden an den Extremitäten.

Die vorliegende Offenbarung betrifft auch die Verwendung einer erfindungsgemäßen Knieorthese zur Behandlung von Knieleiden, insbesondere einer Kniegelenksarthrose, auch Gonarthrose genannt. Die Gonarthrose bezeichnet einen Verschleiß der knorpeligen Gelenkflächen des Kniegelenks. Wenn das mediale oder innere Kompartiment des Femorotibial-Gelenks betroffen ist, spricht man von einer medialen Gonarthrose. Wenn der Patient gleichzeitig unter O-Beinen leidet, liegt eine Varusgonarthrose vor. Ist das laterale oder äußere Kompartiment des Femorotibial-Gelenks betroffen, spricht man von einer lateralen Gonarthrose. Hat der Patient gleichzeitig ein X-Bein, liegt eine Valgusgonarthrose vor. Bevorzugt wird die erfindungsgemäße Knieorthese bei einer medialen Gonarthrose verwendet, insbesondere zur Entlastung des inneren Kompartiments des Femorotibial-Gelenks und zur Schmerzreduzierung.

Die vorliegende Offenbarung betrifft auch ein Behandlungsverfahren zur Behandlung von Gonarthrosen, insbesondere medialen Gonarthrosen, bei dem eine erfindungsgemäße Knieorthese so an das Bein eines Patienten angelegt wird, dass die beiden Kreuzungsbereiche auf der Innenseite des Beins an dem Bein anliegen und das Kopplungselement auf der Außenseite des Beins an dem Bein anliegen.

Die vorliegende Offenbarung betrifft auch ein Behandlungsverfahren zur Behandlung von Leiden der Extremitäten, bei dem eine erfindungsgemäße Extremitätenorthese so an die Extremität eines Patienten angelegt wird, dass die beiden Kreuzungsbereiche auf der Innenseite der Extremität an der Extremität anliegen und das Kopplungselement auf der Außenseite der Extremität an der Extremität anliegen.

Die Erfindung wird anhand eines Beispiels und der Figuren näher erläutert, wobei diese nicht einschränkend zu verstehen sind.
Figur 1 zeigt eine erfindungsgemäße Knieorthese in Vorderansicht;
Figur 2a zeigt die Außenseite Knieorthese von Figur 1;
Figur 2b zeigt einen Detailausschnitt von Figur 2a;
Figur 3 zeigt die Innenseite Knieorthese von Figur 1;
Figur 4 zeigt eine schematische Zeichnung der Kräftewirkung der erfindungsgemäßen Knieorthese;
Figur 5 zeigt eine weitere erfindungsgemäße Knieorthese in Vorderansicht;
Figur 6 zeigt die Außenseite Knieorthese von Figur 5;
Figur 7 zeigt die Innenseite Knieorthese von Figur 5;
Figur 8 zeigt eine erfindungsgemäße Ellenbogenorthese in Vorderansicht;
Figur 9 zeigt die Ellenbogenorthese von Figur 8 bei gekrümmtem Arm;
Figur 10 zeigt die Innenseite Ellenbogenorthese von Figur 8;
Figur 11 zeigt eine weitere erfindungsgemäße Ellenbogenorthese in Vorderansicht;
Figur 12 zeigt die Ellenbogenorthese von Figur 11 bei gekrümmtem Arm;
Figur 13 zeigt die Innenseite Ellenbogenorthese von Figur 11;

### BEISPIELE:

### Knieorthese:

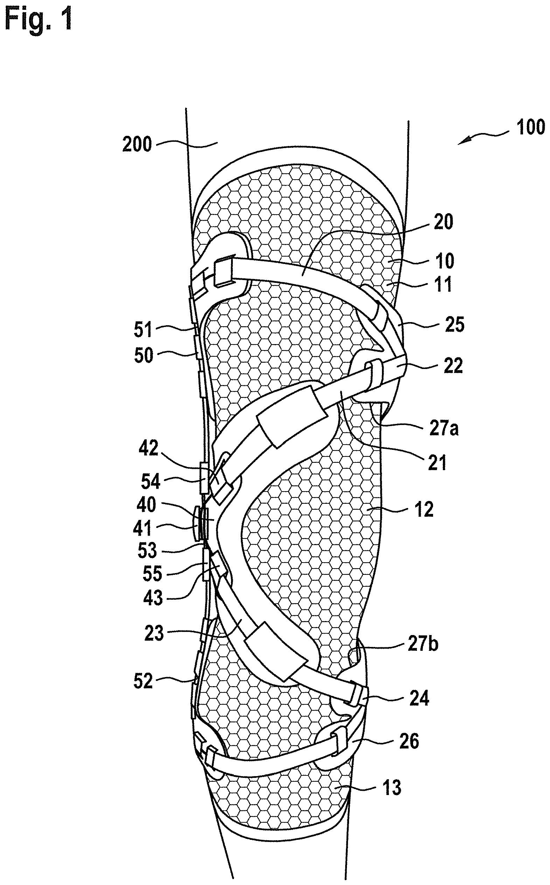

Figur 1 zeigt beispielhaft eine erfindungsgemäße Knieorthese (100) in Vorderansicht an einem Bein (200), mit einer textilen Bandage als Trägerelement (10) mit einem oberen Teilbereich (11), einem mittleren Teilbereich (12) und einem unteren Teilbereich (13). An das Trägerelement (10) ist über ein Kopplungselement (40) ein Gurtsystem (20) gekoppelt, wobei ein erster Gurt als erster Abschnitt (21) des Gurtsystems (20) um den oberen Teilbereich (11) des Trägerelements (10) so geführt ist, dass der Gurt sich in einem ersten Kreuzungsbereich (22) kreuzt und wobei ein zweiter Gurt als zweiter Abschnitt (23) des Gurtsystems (20) um den unteren Teilbereich (13) des Trägerelements (10) so geführt ist, dass der Gurt sich in einem zweiten Kreuzungsbereich (24) kreuzt. Beide Abschnitte (21,23) des Gurtsystems (20) sind dabei jeweils als Schlinge ausgebildet, umschlingen also den jeweiligen Bereich des Beins (200).

Das Kopplungselement (40) ist am mittleren Teilbereich (12) an dem Trägerelement (10) befestigt und das Gurtsystem (20) ist an dem Kopplungselement (40) über Aufhängungen (42,43) beweglich befestigt. Das Kopplungselement (40) weist einen Drehknopf (41) als Spannelement zum Spannen des Gurtes des ersten Abschnitts (21) des Gurtsystems (20) und zum gleichzeitigen Spannen des Gurtes des zweiten Abschnitts (23) des Gurtsystems (20) auf. Das Spannen erfolgt durch ein Aufwickeln von mit den Gurten verbundenen Seilen auf eine Spule im Drehknopf (40).

Die Gurte (21,23) des Gurtsystems (20) werden aus Schnüren gebildet, die in Flachband-Tunneln verlaufen. In den Figuren sind primär diese Flachbandtunnel des Gurtsystems (20) zu sehen und nicht die darin verlaufenden spannbaren Schnüre.

Das Gurtsystem (20) weist ein erstes Kreuzungselement (25), in dem der Gurt im ersten Kreuzungsbereich (22) geführt wird und ein zweites Kreuzungselement (26) auf, in dem der Gurt im zweiten Kreuzungsbereich (24) geführt wird, wobei das erste Kreuzungselement (25) über eine Klettverbindungen (27a) reversibel an dem oberen Teilbereich (11) des Trägerelements (10) befestigt ist und das das zweite Kreuzungselement (26) über eine Klettverbindungen (27b) reversibel an dem unteren Teilbereich (13) des Trägerelements (10) befestigt ist. Die Kreuzungsbereiche (22,24) sind auf dem auf der Beininnenseite anliegenden Bereich der Bandage (10) positioniert.

Die Knieorthese (100) hat zusätzlich eine Gelenkschiene (50), die auf der Bandage (10) im Bereich der Beinaußenseite angebracht ist. Die Gelenkschiene (50) hat einen ersten Schienenabschnitt (51) im Bereich des oberen Teilbereichs (11) des Trägerelements (10), einen zweiten Schienenabschnitt (52) im Bereich des unteren Teilbereichs (13) des Trägerelements (10) und einen dritten Schienenabschnitt (53) im Bereich des mittleren Teilbereichs (12) des Trägerelements (10). Der erste Schienenabschnitt (51) und der zweite Schienenabschnitt (52) sind jeweils durch ein monozentrisches Gelenk (54,55) mit dem dritten Schienenabschnitt (53) verbunden. Der dritte Schienenabschnitt (53) ist als Federelement in Form einer Blattfeder ausgebildet. Das Kopplungselement (40) ist am mittleren Teilbereich (12) befestigt, so dass das Gurtsystem (20) beim Spannen der Gurte eine Zugkraft auf diesen Bereich der Gelenkschiene (50) ausübt. Das Gurtsystem (20) fixiert die Gelenkschiene (50) ebenfalls an ihrem oberen und unteren Ende.

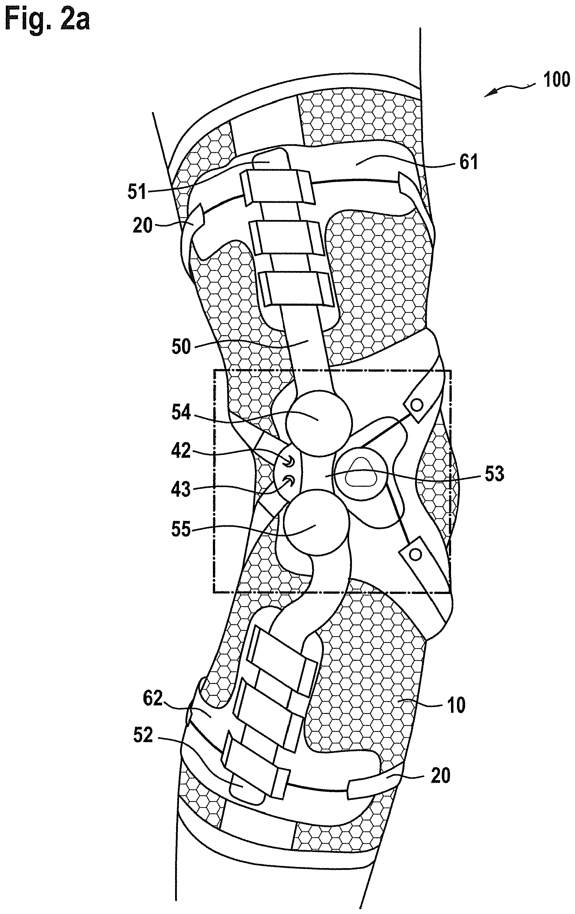

Figur 2a zeigt die Außenseite der Knieorthese (100) von Figur 1. Deutlich zu sehen ist die Bandage (10) mit der anliegenden Gelenkschiene (50), die wieder in die drei Teilbereiche (51,52,53) aufgeteilt ist und die durch die zwei monozentrischen Drehgelenke (54,55) miteinander verbunden sind. Oben und unten ist das Gurtsystem (20) über Schalenelemente (61,62) mit der Gelenkschiene (50) verbunden.

Die Gurte des Gurtsystems (20) sind über bewegliche Aufhängungen (42, 43) mit dem Kopplungselement verbunden.

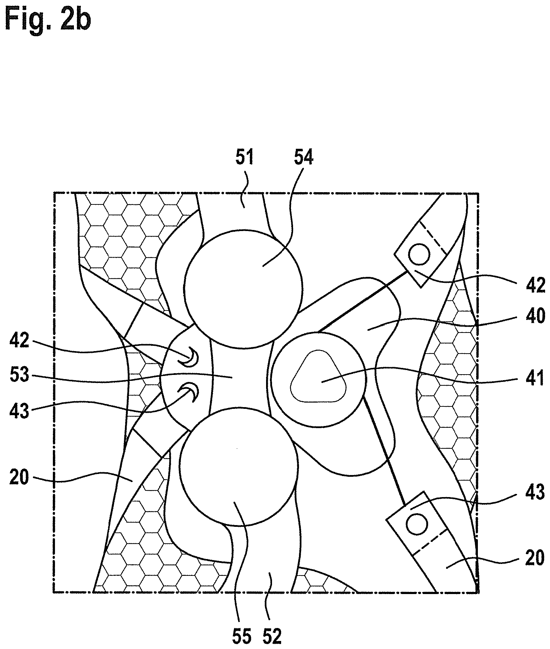

Figur 2b zeigt einen vergrößerten Ausschnitt des dritten Schienenabschnitts (53) aus Figur 2a. Der dritte, mittlere Schienenabschnitt ist als Blattfeder (53) ausgebildet und über die zwei Drehgelenke (54,55) mit den zwei weiteren Abschnitten (51,52) der Gelenkschiene verbunden. An dem mittleren Schienenabschnitt (53) befindet sich das Kopplungselement (40) mit dem Drehknopf (41) als Spannelement. Die Gurte des Gurtsystems (20) sind über bewegliche Aufhängungen (42, 43) mit dem Kopplungselement (40) verbunden.

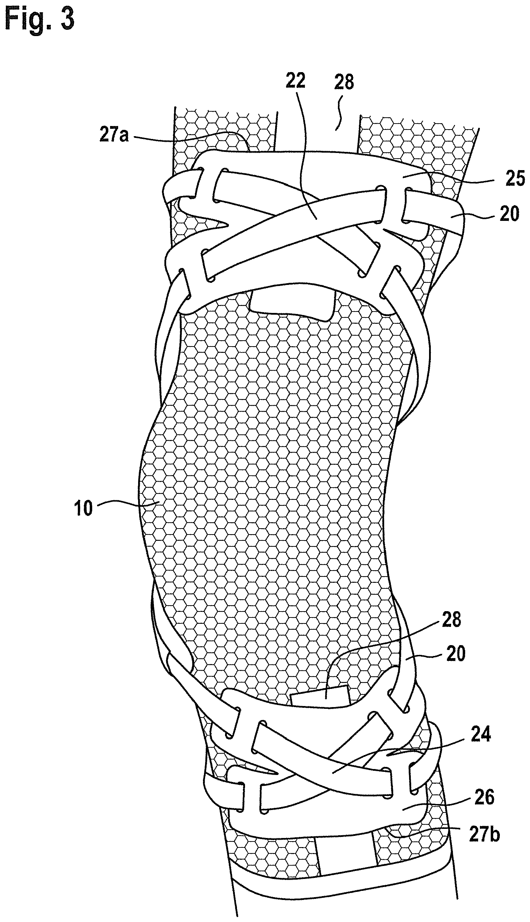

Figur 3 zeigt die Innenseite der Knieorthese von Figur 1. Deutlich zu sehen ist die Bandage (10) mit den Kreuzungsbereichen (22,24) des Gurtsystems (20), die in den Kreuzungselementen (25,26) geführt werden. Die Kreuzungselemente (25,26) sind über unterseitige Klettverbindungen (27a, 27b) flexibel und repositionierbar an Flauschbereichen (28) der Bandage (10) befestigt. Durch die zwei Kreuzungsbereiche (22,24) entsteht eine Gurtführung des Gurtsystems (20) in Form einer doppelten 8.

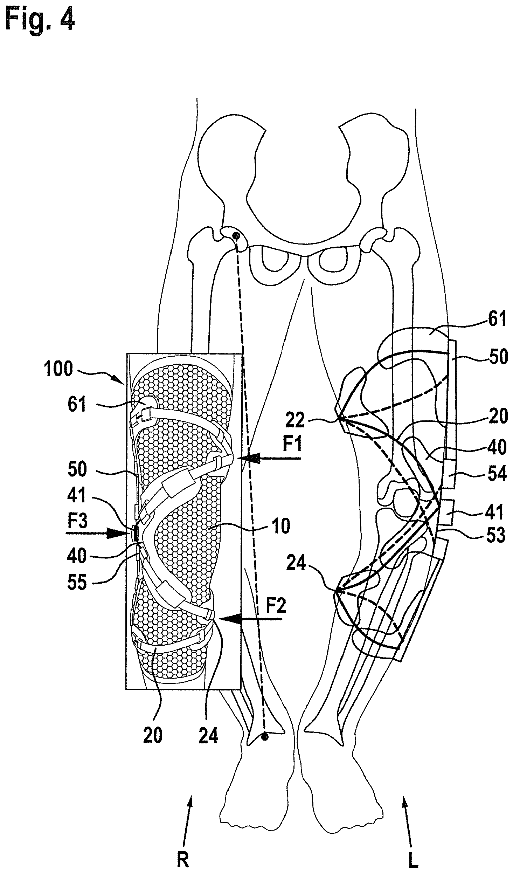

Figur 4 zeigt eine schematische Zeichnung der Kräftewirkung der erfindungsgemäßen Knieorthese (100) bei einer O-Bein Fehlstellung. Am rechten Bein (R) ist die Knieorthese (100) aus Figur 1 zusehen, am linken Bein (L) eine schematische Zeichnung mit wichtigen Teilelementen. Zu sehen ist auf beiden Seiten das Gurtsystem (20) mit den Kreuzungsbereichen (22,24), das Kopplungselement (40) mit Drehknopf (41) zum Spannen des Gurtsystems (20), ein Schalenelement (61) und die Gelenkschiene (50), die wieder in die drei Teilbereiche aufgeteilt ist, die durch die zwei monozentrischen Drehgelenke (54,55) miteinander verbunden sind. Am linken Bein (L) ist zu sehen wie der federnd ausgeführte mittlere Schienenabschnitt (53) durch die O-Bein Stellung federnd gekrümmt wird. Durch den doppelt gekreuzten Verlauf des Gurtsystems (20) wird die Gelenkschiene (50) besonders gut auf der Außenseite des Beins an das Trägerelement (10) und somit das Bein positionsstabil angepresst, da die beiden innenliegenden Kreuzungsbereiche (22,24) als nach außen wirkende Gegenkräfte (F1, F2) zur nach innen wirkenden Kraft (F3) des Kopplungselements (40) wirken, das an der Gelenkschiene (50) befestigt ist. Somit wirkt die Knieorthese (100) biomechanisch nach dem Dreikräfteprinzip.

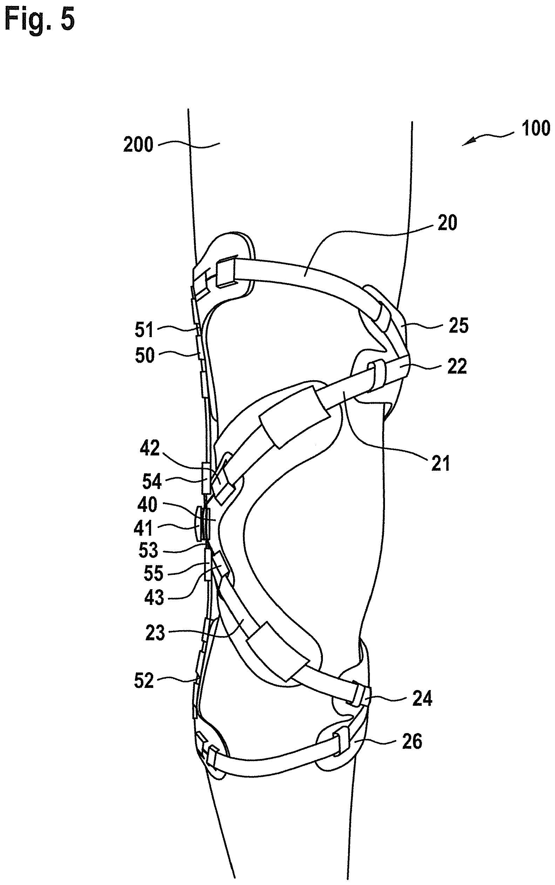

Figur 5 zeigt beispielhaft eine erfindungsgemäße Knieorthese (100) mit einem Gurtsystem (20) wie aus Figur 1 in Vorderansicht an einem Bein (200), jedoch in einer Ausführungsform ohne Trägerelement. Das Gurtsystem (20) ist an ein Kopplungselement (40) gekoppelt, wobei ein erster Gurt als erster Abschnitt (21) des Gurtsystems (20) so geführt ist, dass der Gurt sich in einem ersten Kreuzungsbereich (22) kreuzt und wobei ein zweiter Gurt als zweiter Abschnitt (23) des Gurtsystems (20) so geführt ist, dass der Gurt sich in einem zweiten Kreuzungsbereich (24) kreuzt.

Das Gurtsystem (20) ist an dem Kopplungselement (40) über Aufhängungen (42,43) beweglich befestigt. Das Kopplungselement (40) weist einen Drehknopf (41) als Spannelement zum Spannen des Gurtes des ersten Abschnitts (21) des Gurtsystems (20) und zum gleichzeitigen Spannen des Gurtes des zweiten Abschnitts (23) des Gurtsystems (20) auf. Das Spannen erfolgt durch ein Aufwickeln von mit den Gurten verbundenen Seilen auf eine Spule im Drehknopf (40).

Die Gurte (21,23) des Gurtsystems (20) werden aus Schnüren gebildet, die in Flachband-Tunneln verlaufen. In den Figuren sind primär diese Flachbandtunnel des Gurtsystems (20) zu sehen und nicht die darin verlaufenden spannbaren Schnüre.

Das Gurtsystem (20) weist ein erstes Kreuzungselement (25), in dem der Gurt im ersten Kreuzungsbereich (22) geführt wird und ein zweites Kreuzungselement (26) auf, in dem der Gurt im zweiten Kreuzungsbereich (24) geführt wird, Die Kreuzungsbereiche (22,24) sind auf dem auf der Beininnenseite positioniert.

Die Knieorthese (100) hat zusätzlich eine Gelenkschiene (50), die im Bereich der Beinaußenseite angebracht ist. Die Gelenkschiene (50) hat einen ersten Schienenabschnitt (51), einen zweiten Schienenabschnitt (52) und einen dritten Schienenabschnitt (53). Der erste Schienenabschnitt (51) und der zweite Schienenabschnitt (52) sind jeweils durch ein monozentrisches Gelenk (54,55) mit dem dritten Schienenabschnitt (53) verbunden. Der dritte Schienenabschnitt (53) ist als Federelement in Form einer Blattfeder ausgebildet. Das Kopplungselement (40) ist am Gurtsystem (20) und der Gelenkschiene (50) befestigt, so dass das Gurtsystem (20) beim Spannen der Gurte eine Zugkraft auf diesen Bereich der Gelenkschiene (50) ausübt. Das Gurtsystem (20) fixiert die Gelenkschiene (50) ebenfalls an ihrem oberen und unteren Ende.

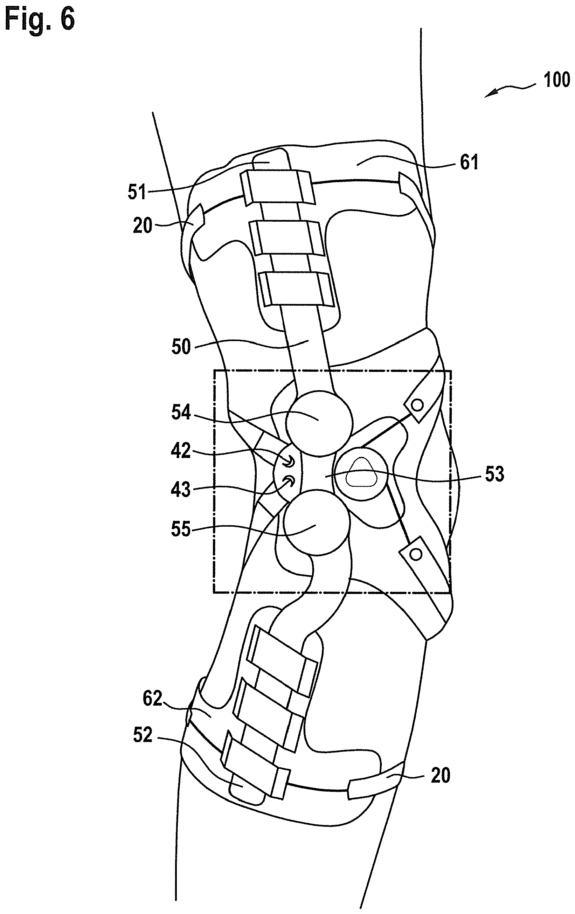

Figur 6 zeigt die Außenseite der Knieorthese (100) von Figur 5. Deutlich zu sehen ist die Gelenkschiene (50), die wieder in die drei Teilbereiche (51,52,53) aufgeteilt ist und die durch die zwei monozentrischen Drehgelenke (54,55) miteinander verbunden sind. Oben und unten ist das Gurtsystem (20) über Schalenelemente (61,62) mit der Gelenkschiene (50) verbunden.

Die Gurte des Gurtsystems (20) sind über bewegliche Aufhängungen (42, 43) mit dem Kopplungselement verbunden.

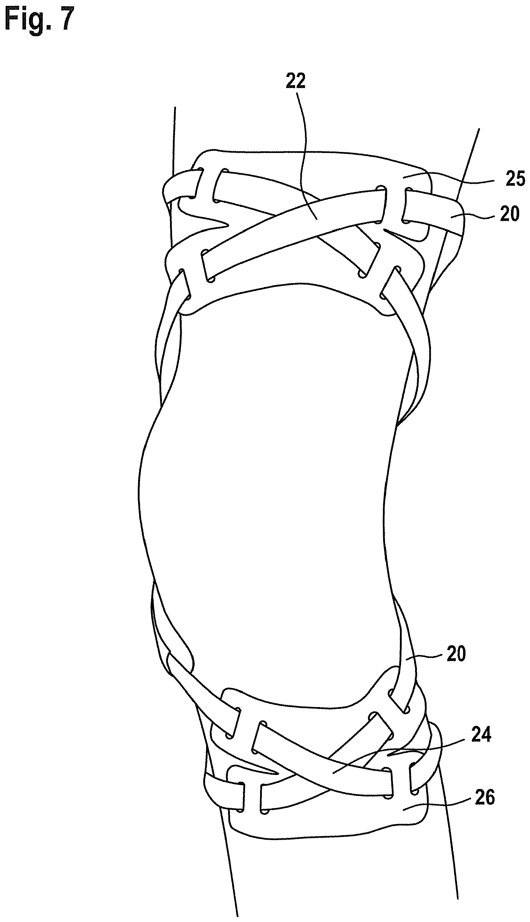

Figur 7 zeigt die Innenseite der Knieorthese von Figur 5. Deutlich zu sehen sind die Kreuzungsbereiche (22,24) des Gurtsystems (20), die in den Kreuzungselementen (25,26) geführt werden. Durch die zwei Kreuzungsbereiche (22,24) entsteht eine Gurtführung des Gurtsystems (20) in Form einer doppelten 8.

### Ellenbogenorthese:

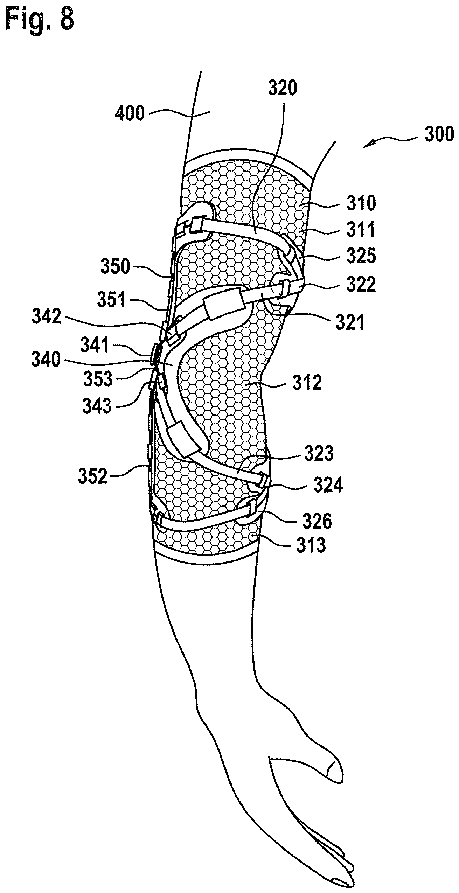

Figur 8 zeigt beispielhaft eine erfindungsgemäße Ellenbogenorthese (300) in Vorderansicht an einem Arm (400), mit einer textilen Bandage als Trägerelement (310) mit einem oberen Teilbereich (311), einem mittleren Teilbereich (312) und einem unteren Teilbereich (313). An das Trägerelement (310) ist über ein Kopplungselement (340) ein Gurtsystem (320) gekoppelt, wobei ein erster Gurt als erster Abschnitt (321) des Gurtsystems (320) um den oberen Teilbereich (311) des Trägerelements (310) so geführt ist, dass der Gurt sich in einem ersten Kreuzungsbereich (322) kreuzt und wobei ein zweiter Gurt als zweiter Abschnitt (323) des Gurtsystems (320) um den unteren Teilbereich (313) des Trägerelements (310) so geführt ist, dass der Gurt sich in einem zweiten Kreuzungsbereich (324) kreuzt. Beide Abschnitte (321,323) des Gurtsystems (320) sind dabei jeweils als Schlinge ausgebildet, umschlingen also den jeweiligen Bereich des Arms (400).

Das Kopplungselement (340) ist am mittleren Teilbereich (312) an dem Trägerelement (310) befestigt und das Gurtsystem (320) ist an dem Kopplungselement (340) über Aufhängungen (342,343) beweglich befestigt. Das Kopplungselement (340) weist einen Drehknopf (341) als Spannelement zum Spannen des Gurtes des ersten Abschnitts (321) des Gurtsystems (320) und zum gleichzeitigen Spannen des Gurtes des zweiten Abschnitts (323) des Gurtsystems (320) auf. Das Spannen erfolgt durch ein Aufwickeln von mit den Gurten verbundenen Seilen auf eine Spule im Drehknopf (340).

Die Gurte (321,323) des Gurtsystems (320) werden aus Schnüren gebildet, die in Flachband-Tunneln verlaufen. In den Figuren sind primär diese Flachbandtunnel des Gurtsystems (320) zu sehen und nicht die darin verlaufenden spannbaren Schnüre.

Das Gurtsystem (320) weist ein erstes Kreuzungselement (325), in dem der Gurt im ersten Kreuzungsbereich (322) geführt wird und ein zweites Kreuzungselement (326) auf, in dem der Gurt im zweiten Kreuzungsbereich (324) geführt wird.

Die Ellenbogenorthese (300) hat zusätzlich eine Gelenkschiene (350), die auf der Bandage (310) im Bereich der Beinaußenseite angebracht ist. Die Gelenkschiene (350) hat einen ersten Schienenabschnitt (351) im Bereich des oberen Teilbereichs (311) des Trägerelements (310), einen zweiten Schienenabschnitt (352) im Bereich des unteren Teilbereichs (313) des Trägerelements (310) und einen dritten Schienenabschnitt (353) im Bereich des mittleren Teilbereichs (312) des Trägerelements (310). Der erste Schienenabschnitt (351) und der zweite Schienenabschnitt (352) sind jeweils durch ein monozentrisches Gelenk mit dem dritten Schienenabschnitt (353) verbunden. Der dritte Schienenabschnitt (353) ist als Federelement in Form einer Blattfeder ausgebildet. Das Kopplungselement (340) ist am mittleren Teilbereich (312) befestigt, so dass das Gurtsystem (320) beim Spannen der Gurte eine Zugkraft auf diesen Bereich der Gelenkschiene (350) ausübt. Das Gurtsystem (320) fixiert die Gelenkschiene (350) ebenfalls an ihrem oberen und unteren Ende.

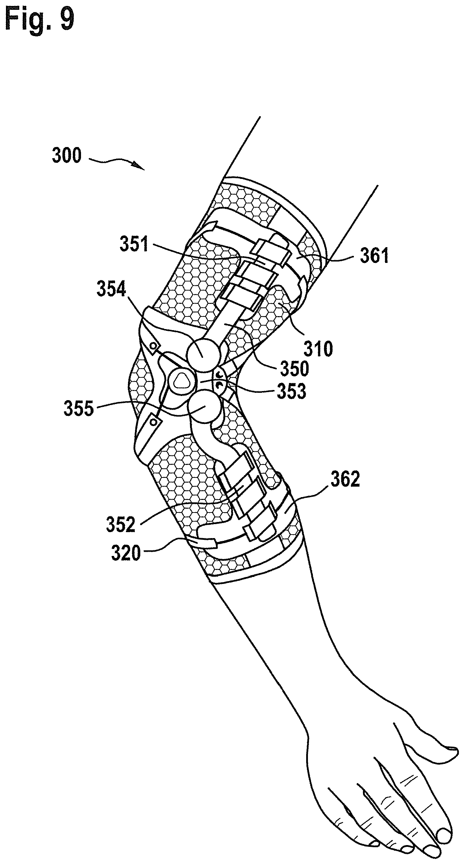

Figur 9 zeigt die Außenseite der Ellenbogenorthese (300) von Figur 8. Deutlich zu sehen ist die Bandage (310) mit der anliegenden Gelenkschiene (350), die wieder in die drei Teilbereiche (351,352,353) aufgeteilt ist und die durch die zwei monozentrischen Drehgelenke (354,355) miteinander verbunden sind. Oben und unten ist das Gurtsystem (320) über Schalenelemente (361,362) mit der Gelenkschiene (350) verbunden.

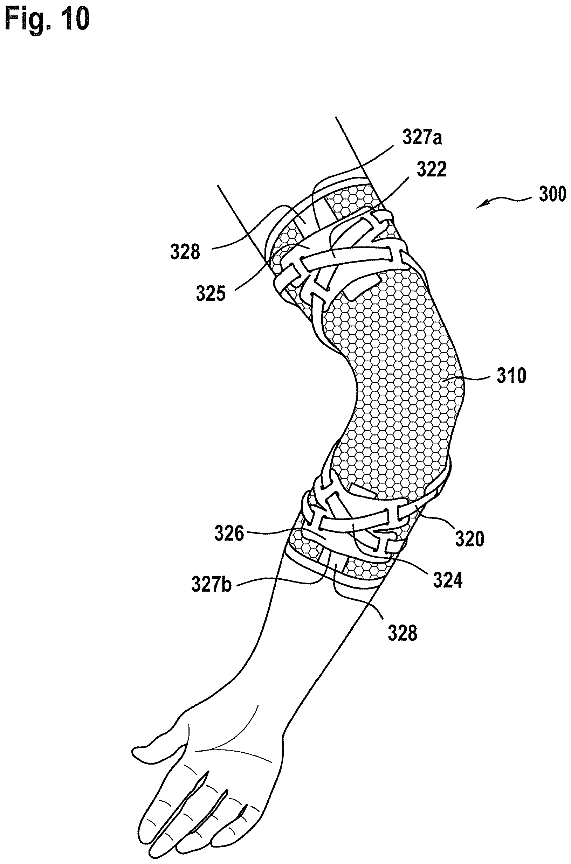

Figur 10 zeigt die Innenseite der Ellenbogenorthese (300) von Figur 8. Deutlich zu sehen ist die Bandage (310) mit den Kreuzungsbereichen (322,324) des Gurtsystems (320), die in den Kreuzungselementen (325,326) geführt werden. Die Kreuzungselemente (325,326) sind über unterseitige Klettverbindungen (327a, 327b) flexibel und repositionierbar an Flauschbereichen (328) der Bandage (310) befestigt. Durch die zwei Kreuzungsbereiche (322,324) entsteht eine Gurtführung des Gurtsystems (320) in Form einer doppelten 8.

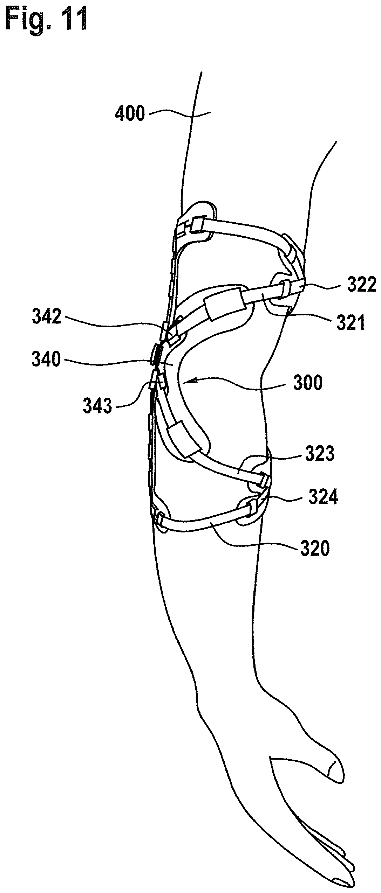

Figur 11 zeigt beispielhaft eine erfindungsgemäße Ellenbogenorthese (300) mit einem Gurtsystem (320) wie aus Figur 8 in Vorderansicht an einem Arm (400), jedoch in einer Ausführungsform ohne Trägerelement. Das Gurtsystem (320) ist an ein Kopplungselement (340) gekoppelt, wobei ein erster Gurt als erster Abschnitt (321) des Gurtsystems (320) so geführt ist, dass der Gurt sich in einem ersten Kreuzungsbereich (322) kreuzt und wobei ein zweiter Gurt als zweiter Abschnitt (323) des Gurtsystems (320) so geführt ist, dass der Gurt sich in einem zweiten Kreuzungsbereich (324) kreuzt.

Das Gurtsystem (320) ist an dem Kopplungselement (340) über Aufhängungen (342,343) beweglich befestigt. Zum weiteren Aufbau der Orthese (300) wird auf die Beschreibung der Figuren 5 und 8 verwiesen.

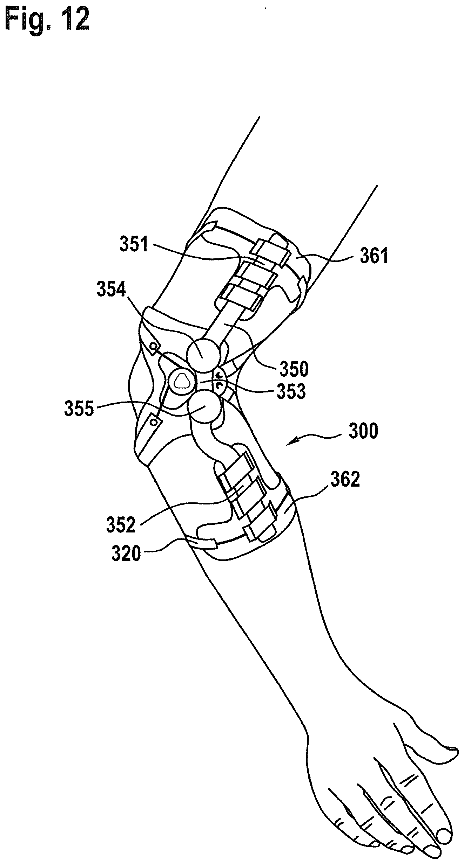

Figur 12 zeigt die Außenseite der Ellenbogenorthese (300) von Figur 11. Deutlich zu sehen ist die Gelenkschiene (350), die wieder in die drei Teilbereiche (351,352,353) aufgeteilt ist und die durch die zwei monozentrischen Drehgelenke (354,355) miteinander verbunden sind. Oben und unten ist das Gurtsystem (320) über Schalenelemente (361,362) mit der Gelenkschiene (350) verbunden.

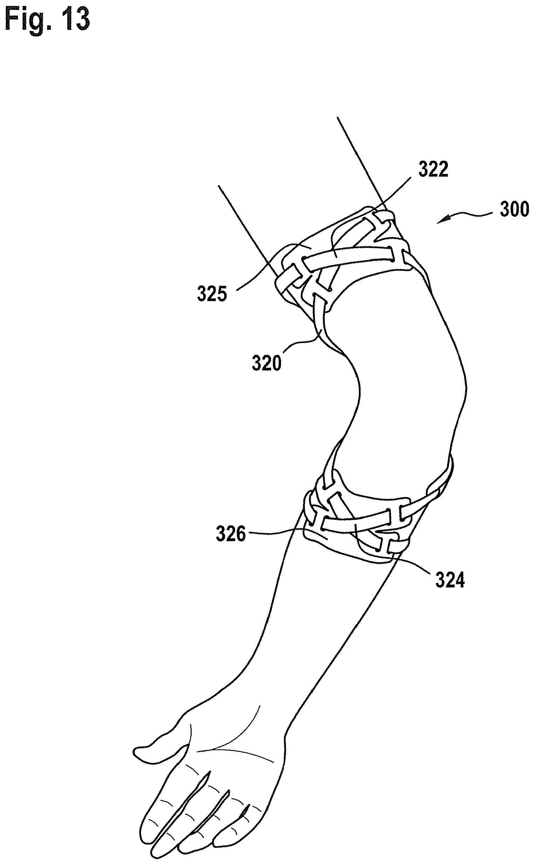

Figur 13 zeigt die Innenseite der Ellenbogenorthese (300) von Figur 11. Deutlich zu sehen sind die Kreuzungsbereiche (322,324) des Gurtsystems (320), die in den Kreuzungselementen (325,326) geführt werden. Durch die zwei Kreuzungsbereiche (322,324) entsteht eine Gurtführung des Gurtsystems (320) in Form einer doppelten 8.

## Patentansprüche

1. Extremitätenorthese (100, 300), umfassend ein Gurtsystem (20), **dadurch gekennzeichnet, dass** ein Gurt als erster Abschnitt (21) des Gurtsystems (20) so geführt ist, dass der Gurt sich in einem ersten Kreuzungsbereich (22) kreuzt und dass ein Gurt als zweiter Abschnitt (23) des Gurtsystems (20) so geführt ist, dass der Gurt sich in einem zweiten Kreuzungsbereich (24) kreuzt und umfassend ein Kopplungselement (40), an dem der erste Abschnitt (21) des Gurtsystems (20) und der zweite Abschnitt (23) des Gurtsystems (20) gekoppelt sind, wobei bei der angelegten Extremitätenorthese (100, 300) der erste Abschnitt (21) und der zweite Abschnitt (23) des Gurtsystems (20) so um die Extremität geführt sind, dass der erste Abschnitt (21) und der zweite Abschnitt (22) des Gurtsystems (20) die Extremität umschlingt und sich dabei mindestens einmal kreuzt, wobei das Kopplungselement im angelegten Zustand auf Höhe des Knies am Bein oder auf Höhe des Ellenbogens am Arm anliegt.

2. Extremitätenorthese (100, 300) nach Anspruch 1, wobei die Extremität ein Bein ist.

3. Extremitätenorthese nach einem der vorhergehenden Ansprüche, wobei das Kopplungselement im angezogenen Zustand der Extremitätenorthese auf der Außenseite der Extremität positioniert ist.

4. Extremitätenorthese (100, 300) nach einem der vorhergehenden Ansprüche, umfassend ein Trägerelement (10) mit einem oberen Teilbereich (11), einem mittleren Teilbereich (12) und einem unteren Teilbereich (13) und umfassend das an das Trägerelement (10) über das Kopplungselement (40) gekoppelte Gurtsystem (20), wobei ein Gurt als erster Abschnitt (21) des Gurtsystems (20) um den oberen Teilbereich (11) des Trägerelements (10) so geführt ist, dass der Gurt sich in dem ersten Kreuzungsbereich (22) kreuzt und wobei ein Gurt als zweiter Abschnitt (23) des Gurtsystems (20) um den unteren Teilbereich (13) des Trägerelements (10) so geführt ist, dass der Gurt sich in dem zweiten Kreuzungsbereich (24) kreuzt.

5. Extremitätenorthese nach Anspruch 4, wobei das Trägerelement (10) eine Bandage ist.

6. Extremitätenorthese nach einem der vorhergehenden Ansprüche, wobei die Extremitätenorthese eine Knieorthese (100) ist.

7. Extremitätenorthese nach einem der Ansprüche 4-6, wobei das Kopplungselement (40) am mittleren Teilbereich (12) an dem Trägerelement (10) befestigt ist.

8. Extremitätenorthese nach einem der Ansprüche 4-7, wobei das Gurtsystem (20) ein erstes Kreuzungselement (25) aufweist, in dem der Gurt im ersten Kreuzungsbereich (22) geführt wird und ein zweites Kreuzungselement (26) aufweist, in dem der Gurt im zweiten Kreuzungsbereich (24) geführt wird, wobei bevorzugt das erste Kreuzungselement (25) reversibel an dem oberen Teilbereich (11) des Trägerelements (10) befestigbar ist und bevorzugt das das zweite Kreuzungselement (26) reversibel an dem unteren Teilbereich (13) des Trägerelements (10) befestigbar ist.

9. Extremitätenorthese nach Anspruch 8, wobei das erste (25) und das zweite (26) Kreuzungselement über Klettverbindungen (27,28) an verschiedenen Stellen des Trägerelements (10) reversibel befestigbar sind.

10. Extremitätenorthese nach einem der vorhergehenden Ansprüche, wobei der erste Abschnitt (21) des Gurtsystems (20) durch einen ersten Gurt gebildet wird und der zweite Abschnitt (23) des Gurtsystems (20) durch einen zweiten Gurt gebildet wird.

11. Extremitätenorthese nach einem der vorhergehenden Ansprüche, wobei das Gurtsystem (20) an dem Kopplungselement (40) beweglich befestigt ist.

12. Extremitätenorthese nach einem der vorhergehenden Ansprüche, wobei das Kopplungselement ein Spannelement (41) zum Spannen des Gurtes des ersten Abschnitts (21) des Gurtsystems (20) und/oder zum Spannen des Gurtes des zweiten Abschnitts (23) des Gurtsystems (20) aufweist.

13. Extremitätenorthese nach einem der vorhergehenden Ansprüche, wobei die Extremitätenorthese (100) eine Gelenkschiene (50) aufweist.

14. Extremitätenorthese nach Anspruch 13, wobei die Gelenkschiene (50) einen ersten Schienenabschnitt (51), einen zweiten Schienenabschnitt (52) und einen dritten Schienenabschnitt (53) aufweist, und wobei der erste Schienenabschnitt (51) und der zweite Schienenabschnitt (52) jeweils durch ein Gelenk (54,55) mit dem dritten Schienenabschnitt (53) verbunden sind.

15. Extremitätenorthese nach Anspruch 13, wobei die Gelenkschiene (50) einen ersten Schienenabschnitt (51) im Bereich des oberen Teilbereichs (11) des Trägerelements (10), einen zweiten Schienenabschnitt (52) im Bereich des unteren Teilbereichs (13) des Trägerelements (10) und einen dritten Schienenabschnitt (53) im Bereich des mittleren Teilbereichs (12) des Trägerelements (10) aufweist, und wobei der erste Schienenabschnitt (51) und der zweite Schienenabschnitt (52) jeweils durch ein Gelenk (54,55) mit dem dritten Schienenabschnitt (53) verbunden sind.

16. Extremitätenorthese nach einem der Ansprüche 14 oder 15, wobei der dritte Schienenabschnitt (53) aus einem Federelement, insbesondere einer Blattfeder, gebildet wird.

## Claims

1. Limb orthosis (100, 300), comprising a strap system (20), **characterized in that** a strap as a first section (21) of the strap system (20) is routed in such a manner that the strap crosses over in a first crossover region (22) and that a strap as a second section (23) of the strap system (20) is routed in such a manner that the strap crosses over in a second crossover region (24), and comprising a coupling element (40) to which the first section (21) of the strap system (20) and the second section (23) of the strap system (20) are coupled, wherein for the donned limb orthosis (100, 300), the first section (21) and the second section (23) of the strap system (20) are routed around the limb in such a manner that the first section (21) and the second section (22) of the strap system (20) wrap around the limb and thereby cross over at least once, wherein the coupling element in the donned state contacts the leg at knee level or contacts the arm at elbow level.

2. Limb orthosis (100, 300) according to claim 1, wherein the limb is a leg.

3. Limb orthosis according to any one of the preceding claims, wherein the coupling element in the donned state of the limb orthosis is positioned on the outside of the limb.

4. Limb orthosis (100, 300) according to any one of the preceding claims, comprising a carrier element (10) having an upper subregion (11), a central subregion (12) and a lower subregion (13) and comprising the strap system (20) coupled to the carrier element (10) by means of the coupling element (40), wherein a strap as a first section (21) of the strap system (20) is routed around the upper subregion (11) of the carrier element (10) in such a manner that the strap crosses over in the first crossover region (22) and wherein a strap as a second section (23) of the strap system (20) is routed around the lower subregion (13) of the carrier element (10) in such a manner that the strap crosses over in the second crossover region (24).

5. Limb orthosis according to claim 4, wherein the carrier element (10) is a support.

6. Limb orthosis according to any one of the preceding claims, wherein the limb orthosis is a knee orthosis (100).

7. Limb orthosis according to any one of claims 4-6, wherein the coupling element (40) is attached to the central subregion (12) on the carrier element (10).

8. Limb orthosis according to any one of claims 4-7, wherein the strap system (20) has a first crossing element (25) in which the strap is routed in a first crossover region (22) and has a second crossing element (26) in which the strap is routed in a second crossover region (24), wherein preferably the first crossing element (25) can be reversibly attached to the upper subregion (11) of the carrier element (10) and preferably the second crossing element (26) can be reversibly attached to the lower subregion (13) of the carrier element (10).

9. Limb orthosis according to claim 8, wherein the first (25) and the second (26) crossing element can be reversibly attached by means of hook and loop connections (27, 28) to various points of the carrier element (10).

10. Limb orthosis according to any one of the preceding claims, wherein the first section (21) of the strap system (20) is formed by a first strap and the second section (23) of the strap system (20) is formed by a second strap.

11. Limb orthosis according to any one of the preceding claims, wherein the strap system (20) is movably attached to the coupling element (40).

12. Limb orthosis according to any one of the preceding claims, wherein the coupling element has a tensioning element (41) for tensioning the strap of the first section (21) of the strap system (20) and/or for tensioning the strap of the second section (23) of the strap system (20).

13. Limb orthosis according to any one of the preceding claims, wherein the limb orthosis (100) has a joint splint (50).

14. Limb orthosis according to claim 13, wherein the joint splint (50) has a first bar section (51), a second bar section (52) and a third bar section (53), and wherein the first bar section (51) and the second bar section (52) are each connected by a joint (54, 55) to the third bar section (53).

15. Limb orthosis according to claim 13, wherein the joint splint (50) has a first bar section (51) in the region of the upper subregion (11) of the carrier element (10), a second bar section (52) in the region of the lower subregion (13) of the carrier element (10) and a third bar section (53) in the region of the central subregion (12) of the carrier element (10), and wherein the first bar section (51) and the second bar section (52) are each connected by a joint (54, 55) to the third bar section (53).

16. Limb orthosis according to one of the claims 14 or 15, wherein the third bar section (53) is formed of a spring element, particularly a leaf spring.

## Revendications

1. Orthèse d'extrémité (100, 300), comprenant un système de sangle (20), **caractérisée en ce qu'**une sangle est guidée comme premier segment (21) du système de sangle (20) de telle manière que la sangle se croise dans une première zone de croisement (22) et **en ce qu'**une sangle est guidée comme deuxième segment (23) du système de sangle (20) de telle manière que la sangle se croise dans une deuxième zone de croisement (24) et comprenant un élément de couplage (40), auquel le premier segment (21) du système de sangle (20) et le deuxième segment (23) du système de sangle (20) sont couplés, dans laquelle, lorsque l'orthèse d'extrémité (100, 300) est en place, le premier segment (21) et le deuxième segment (23) du système de sangle (20) sont guidés autour de l'extrémité de telle manière que le premier segment (21) et le deuxième segment (22) du système de sangle (20) enlacent l'extrémité et se croisent alors au moins une fois, l'élément de couplage s'appliquant contre la jambe à la hauteur du genou ou contre le bras à la hauteur du coude lorsque l'orthèse est en place.

2. Orthèse d'extrémité (100, 300) selon la revendication 1, dans laquelle l'extrémité est une jambe.

3. Orthèse d'extrémité selon l'une des revendications précédentes, dans laquelle l'élément de couplage est positionné sur le côté extérieur de l'extrémité lorsque l'orthèse d'extrémité est en place.

4. Orthèse d'extrémité (100, 300) selon l'une des revendications précédentes, comprenant un élément de support (10) doté d'une partie supérieure (11), d'une partie centrale (12) et d'une partie inférieure (13) et comprenant le système de sangle (20) couplé à l'élément de support (10) par le biais de l'élément de couplage (40), une sangle étant guidée comme premier segment (21) du système de sangle (20) autour de la partie supérieure (11) de l'élément de support (10) de telle manière que la sangle se croise dans la première zone de croisement (22) et une sangle étant guidée comme deuxième segment (23) du système de sangle (20) autour de la partie inférieure (13) de l'élément de support (10) de telle manière que la sangle se croise dans la deuxième zone de croisement (24).

5. Orthèse d'extrémité selon la revendication 4, dans laquelle l'élément de support (10) est un bandage.

6. Orthèse d'extrémité selon l'une des revendications précédentes, dans laquelle l'orthèse d'extrémité est une orthèse de genou (100).

7. Orthèse d'extrémité selon l'une des revendications 4 à 6, dans laquelle l'élément de couplage (40) est fixé à l'élément de support (10) sur la partie centrale (12).

8. Orthèse d'extrémité selon l'une des revendications 4 à 7, dans laquelle le système de sangle (20) comporte un premier élément de croisement (25), dans lequel la sangle est guidée dans la première zone de croisement (22) et comporte un deuxième élément de croisement (26), dans lequel la sangle est guidée dans la deuxième zone de croisement (24), le premier élément de croisement (25) pouvant de préférence être fixé de manière réversible à la partie supérieure (11) de l'élément de support (10) et le deuxième élément de croisement (26) pouvant de préférence être fixé de manière réversible à la partie inférieure (13) de l'élément de support (10).

9. Orthèse d'extrémité selon la revendication 8, dans laquelle le premier (25) et le deuxième (26) élément de croisement peuvent être fixés de manière réversible à différents endroits de l'élément de support (10) par le biais d'assemblages par ruban autoagrippant (27, 28).

10. Orthèse d'extrémité selon l'une des revendications précédentes, dans laquelle le premier segment (21) du système de sangle (20) est formé par une première sangle et le deuxième segment (23) du système de sangle (20) est formé par une deuxième sangle.

11. Orthèse d'extrémité selon l'une des revendications précédentes, dans laquelle le système de sangle (20) est fixé à l'élément de couplage (40) de manière mobile.

12. Orthèse d'extrémité selon l'une des revendications précédentes, dans laquelle l'élément de couplage comporte un élément de serrage (41) pour tendre la sangle du premier segment (21) du système de sangle (20) et/ou pour tendre la sangle du deuxième segment (23) du système de sangle (20).

13. Orthèse d'extrémité selon l'une des revendications précédentes, dans laquelle l'orthèse d'extrémité (100) comporte une attelle articulée (50).

14. Orthèse d'extrémité selon la revendication 13, dans laquelle l'attelle articulée (50) comporte un premier segment d'attelle (51), un deuxième segment d'attelle (52) et un troisième segment d'attelle (53), et dans laquelle le premier segment d'attelle (51) et le deuxième segment d'attelle (52) sont respectivement reliés au troisième segment d'attelle (53) par une articulation (54, 55).

15. Orthèse d'extrémité selon la revendication 13, dans laquelle l'attelle articulée (50) comporte un premier segment d'attelle (51) dans la zone de la partie supérieure (11) de l'élément de support (10), un deuxième segment d'attelle (52) dans la zone de la partie inférieure (13) de l'élément de support (10) et un troisième segment d'attelle (53) dans la zone de la partie centrale (12) de l'élément de support (10), et dans laquelle le premier segment d'attelle (51) et le deuxième segment d'attelle (52) sont respectivement reliés au troisième segment d'attelle (53) par une articulation (54, 55).

16. Orthèse d'extrémité selon l'une des revendications 14 ou 15, dans laquelle le troisième segment d'attelle (53) est formé par un élément de ressort, en particulier un ressort à lame.
